# FASCICULE DE BREVET EUROPEEN

(11) **EP 2 301 518 B1**
(45) Date de publication et mention de la délivrance du brevet: **08.08.2012**
(21) Numéro de dépôt: 10172461.5
(22) Date de dépôt: 11.08.2010
(51) Int. Cl.: A61K 8/34, A61K 8/37, A61K 8/81, A61Q 19/00, A61K 8/49, A61K 8/86, A61K 8/90, A61K 8/06

(54) **Composition cosmétique comprenant un dérivé diphényl-méthane hydroxylé**
Kosmetische Zusammensetzung, die ein hydroxyliertes Diphenylmethanderivat enthält
Cosmetic composition including a hydroxylated diphenyl-methane derivative

(30) Priorité: 28.08.2009 FR 0955877
(43) Date de publication de la demande: 30.03.2011
(73) Titulaire: L'Oréal, 75008 Paris (FR)
(72) Inventeur: Monello, Aldo, 91600, Savigny sur Orge (FR); Achram, Mélanie, 75013, Paris (FR)
(74) Mandataire: Le Blainvaux Bellegarde, Françoise

(56) Documents cités:
- EP-A1- 1 847 247
- EP-A1- 1 857 095
- WO-A2-02/19984
- DE-A1- 19 935 763
- US-A1- 2007 269 390
- US-A1- 2009 130 035
- US-A1- 2009 162 305

## Description

La présente invention a pour objet une émulsion huile-dans-eau cosmétique comprenant un dérivé diphényl-méthane hydroxylé et un mélange particulier de tensioactif.

Il est connu d'utiliser des actifs dans des compositions cosmétiques et/ou dermatologiques, par exemple en vue de soigner ou de traiter ou d'apporter des effets bénéfiques à la peau. Toutefois, certains de ces actifs présentent l'inconvénient d'être instables dans les solvants cosmétiques classiques et/ou de se dégrader facilement, notamment au contact de l'eau, en particulier à cause de phénomènes d'oxydation. Ils perdent ainsi rapidement leur activité au cours du temps et cette instabilité va à l'encontre de l'efficacité recherchée.

Les dérivés diphényl-méthane hydroxylés sont connus de la demande US 2007/098655 dans des compositions sous forme d'émulsion. Ces dérivés diphényl-méthane hydroxylés sont décrits dans cette demande comme inhibiteurs de tyrosinase, utilisables notamment dans des compositions dépigmentantes.

Ces dérivés diphényl-méthane hydroxylés, du fait notamment de leur structure aromatique et de leur caractère lipophile, présentent l'inconvénient d'être instables et/ou faiblement solubles dans les solvants classiques utilisés en cosmétique. Ils peuvent notamment se recristalliser. Ils peuvent en outre se dégrader facilement à la lumière et/ou à la température, notamment à cause de phénomènes d'oxydation.
Ils perdent ainsi rapidement leur activité au cours du temps et cette instabilité va à l'encontre de l'efficacité recherchée.

Par ailleurs, l'utilisation de dérivés diphényl-méthane hydroxylés tels que le 4-(1-phényléthyl)-1,3-dihydroxybenzène dans des émulsions, et en particulier dans des émulsions huile-dans-eau, notamment lorsqu'elles contiennent un système tensioactif comprenant un ester d'acide gras et de polyéthylène glycol ; et un tensioactif additionnel choisi parmi les esters d'acide gras en C₁₆-C₂₂ et de sorbitan et les esters d'acide gras en C₁₆-C₂₂ et de glycéryle , a tendance à déstabiliser l'émulsion qui présente alors un déphasage d'huile en surface . Les globules d'huile dispersés dans la phase aqueuse ont un aspect grossier rendant l'émulsion non homogène.

EP 1847247 décrit une émulsion huile-dans-eau à l'exemple 2c comprenant du phényléthylbenzènediol, un ester d'acide gras en C16-C22 et de sorbitan et un ester d'acide gras éthoxylé , qui est stabilisée par la présence de diméthylisosorbide.

US 2007/269390 décrit une émulsion fine huile-dans-eau comprenant un tensioactif siliconé, un monoalcool, un tensioactif non-ionique et un tensioactif anionique.

DE 19935763 décrit une émulsion huile-dans-eau stable comprenant du rétinol ou un ester de rétinol, un système redox comprenant de l'acide ascorbique ou dérivés et un polyphénol de type catéchol, et un émulsionnant stabilisant qui peut par exemple être choisi parmi la liste des 11 familles de tensioactifs. Le système tensioactif selon l'invention n'est pas divulgué ni suggéré.

WO 02/19984 décrit une émulsion contenant de l'acide salicylique, un copolymère acrylate / alkyl C10-C30 acrylate (Pemulen) et un tensioactif non ionique choisi parmi les éthers polymériques tels que les blocs PEO-POP type Poloxamer, et les mélanges laurate ester de sorbitol et les anhydrides sorbitol condensés par oxyde d'éthylène. Le système tensioactif selon l'invention n'est pas divulgué ni suggéré.

Le but de la présente invention est donc de disposer d'une émulsion contenant un dérivé diphényl-méthane hydroxylé et le système tensioactif décrit précédemment, qui soit stable, notamment pendant 24 heures à 55 °C, voire pendant 2 mois à 45 °C.

Les inventeurs ont découvert que la stabilité d'une telle émulsion peut être obtenue en ajoutant un polycondensat d'oxyde d'éthylène et d'oxyde de propylène.

De façon plus précise, l'invention a pour objet une composition sous forme d'émulsion huile-dans-eau comprenant :
- un dérivé diphényl-méthane hydroxylé tel que décrit ci-après ;
- un ester d'acide gras en C₁₆-C₂₂ comportant de 8 à 100 unités d'oxyde éthylène;
- un tensioactif additionnel choisi parmi les esters d'acide gras en C₁₆-C₂₂ et de sorbitan et les esters d'acide gras en C₁₆-C₂₂ et de glycéryle ;
- un polycondensat d'oxyde d'éthylène et d'oxyde de propylène.

L'invention a également pour objet un procédé non thérapeutique de soin ou de maquillage des matières kératiniques comprenant l'application sur les matières kératiniques de la composition définie précédemment.

Les dérivés diphényl-méthane hydroxylés utilisables dans les compositions de l'invention sont décrits dans la demande WO2004/105736.
Ces composés ont la formule (I) suivante : 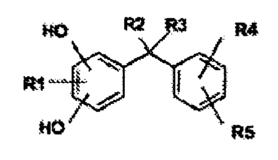 dans laquelle :
- R1 est choisi parmi un atome d'hydrogène, un groupement méthyle, une chaîne alkyle linéaire ou ramifiée, saturée ou insaturée, ayant de 2 à 4 atomes de carbone, un groupement -OH, et un halogène,
- R2 est choisi parmi un atome d'hydrogène, un groupement méthyle, une chaîne alkyle linéaire ou ramifiée, saturée ou insaturée, ayant de 2 à 5 atomes de carbone,
- R3 est choisi parmi un groupement méthyle ou une chaîne alkyle linéaire ou ramifiée, saturée ou insaturée, ayant de 2 à 5 atomes de carbone,
- R4 et R5 sont indépendemment l'un de l'autre choisi parmi un atome d'hydrogène, un groupement méthyle, une chaîne alkyle linéaire ou ramifiée, saturée ou insaturée, ayant de 2 à 5 atomes de carbone, un groupement -OH ou un halogène.

Les groupements -OH, R1, R4 et R5 peuvent être en position ortho, meta ou para par rapport à la liaison formée avec le carbone reliant entre eux les deux noyaux aromatiques.

Sont également compris dans les composés de l'invention possédant les groupes phényl substitués et pour lesquels R2 et R3 sont distincts, les formes énantiomères de configuration S, les énantiomères de configuration R ou leur mélange racémate.

Selon un mode préféré de l'invention, on utilise un composé de formule (I) dans lequel :
- R1, R2, R4 et R5 désignent un atome d'hydrogène ;
- R3 est un groupement méthyle ;
- les groupements -OH sont en position ortho et para par rapport à la liaison formée avec le carbone reliant entre eux entre les deux noyaux aromatiques.

Ce composé correspond à la formule (II) suivante 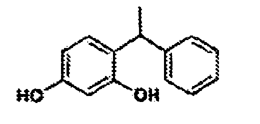 nommé 4-(1-phényléthyl)-1,3-benzenediol ou 4-(1-phényléthyl)-1,3-dihydroxybenzène ou autrement nommé phényléthyl résorcinol ou phénylethylbenzenediol ou styrylrésorcinol. Ce composé a un numéro CAS 85-27-8.

Un tel composé est commercialisé sous la dénomination SYMWHITE 377^{®} ou BIO 377 par la société SYMRISE.

Le composé dérivé diphényl-méthane hydroxylé tel que décrit précédemment peut être présent dans l'émulsion selon l'invention en une teneur allant de 0,01 % à 5 % en poids, par rapport au poids total de la composition, de préférence allant de 0,01 % à 2 % en poids, et préférentiellement allant de 0,01 % à 1 % en poids.

La composition selon l'invention comprend, à titre de tensioactif émulsionnant principal, au moins un ester d'acide gras et de polyéthylène glycol.

L'ester d'acide gras et de polyéthylène glycol présent dans la composition selon l'invention est de préférence un ester d'acides gras en C₁₆-C₂₂ comportant de 8 à 100 unités d'oxyde éthylène.
La chaîne grasse des esters peut être notamment choisie parmi les motifs stéaryle, béhényle, arachidyle, palmityle, cetyle et leurs mélanges tel que cétéaryle, et de préférence une chaîne stéaryle.

Le nombre d'unités d'oxyde d'éthylène peut aller de 8 à 100, de préférence de 10 à 80, et mieux de 10 à 50. Selon un mode particulier de réalisation de l'invention, ce nombre peut aller de 20 à 40.

A titre d'exemple d'ester d'acide gras et de polyéthylène glycol, on peut citer les esters d'acide stéarique comprenant respectivement 20, 30, 40, 50, 100 unités d'oxyde d'éthylène, tels que les produit commercialisés respectivement sous la dénomination Myrj 49 P (stéarate de polyéthylène glycol 20 OE ; nom CTFA: PEG-20 stearate), Myrj 51, Myrj 52 P (stéarate de polyéthylèneglycol 40 OE ; nom CTFA : PEG-40 stearate), Myrj 53, Myrj 59 P par la société CRODA.

L'ester d'acide gras et de polyéthylène glycol peut être présent dans la composition selon l'invention en une teneur allant de 0,1 % à 10 % en poids, par rapport au poids total de la composition, et de préférence allant de 0,1 % à 5 % en poids, et préférentiellement allant de 0,1 % à 3 % en poids.

La composition selon l'invention comprend également un tensioactif émulsionnant additionnel choisi parmi les esters d'acide gras en C₁₆-C₂₂ et de sorbitan et les esters d'acide gras en C₁₆-C₂₂ et de glycéryle.

Selon un premier mode de réalisation de l'invention, la composition comprend un ester d'acide gras en C₁₆-C₂₂ et de sorbitan.

Les esters d'acide gras en C₁₆-C₂₂ et de sorbitan sont formés par estérification d'au moins un acide gras comportant au moins une chaîne alkyle linéaire saturée ou insaturée, ayant respectivement de 16 à 22 atomes de carbone, avec le sorbitol. Ces esters peuvent être notamment choisis parmi les stéarates, béhénates, arachidates, palmitates, oléates de sorbitan, et leurs mélanges. On utilise de préférence des stéarates et palmitates de sorbitan, et préférentiellement les stéarates de sorbitan.
L'ester d'acide gras en C₁₆-C₂₂ et de sorbitan présent dans la composition selon l'invention est avantageusement solide à une température inférieure ou égale à 45°C.

On peut citer à titre d'exemple d'ester de sorbitan utilisable dans composition selon l'invention, le monostéarate de sorbitan (nom CTFA : Sorbitan stearate) vendu par la société Croda sous la dénomination Span 60, le tristéarate de sorbitan vendu par la société Croda sous la dénomination Span 65 V, le monopalmitate de sorbitan (nom CTFA : Sorbitan palmitate) vendu par la société Croda sous la dénomination Span 40, le monoléate de sorbitan vendu par la société Croda sous la dénomination Span 80 V , le trioléate de sorbitan vendu par la société Uniquema sous la dénomination Span 85 V. de préférence, l'ester de sorbitan utilisé est le tristéarate de sorbitan.

L'ester d'acide gras en C₁₆-C₂₂ et de sorbitan peut être présent dans la composition selon l'invention en une teneur allant de 0,01 % à 10 % en poids, par rapport au poids total de la composition, de préférence allant de 0,01 % à 5 % en poids, et préférentiellement allant de 0,1 % à 3 % en poids.

L'ester de glycéryle et d'acide gras peut être obtenu notamment à partir d'un acide comportant une chaîne alkyle linéaire saturée, ayant de 16 à 22 atomes de carbone. Comme ester de glycéryle et d'acide gras, on peut citer notamment le stéarate de glycéryle (mono-, di- et/ou tri-stéarate de glycéryle) (nom CTFA : Glyceryl stearate), le ricinoléate de glycéryle, et leurs mélanges. De préférence, l'ester de glycéryle et d'acide gras utilisé est choisi parmi les stéarates de glycéryle.

L'ester de glycéryle et d'acide gras peut être présent en une quantité allant de 0,1 à 10 % en poids, par rapport au poids total de la composition, de préférence allant de 0,1 à 5 % en poids, et préférentiellement allant de 0,1 % à 3 % en poids.

La composition de l'invention peut comprendre notamment un mélange de stéarate de glycéryle et de monostéarate de polyéthylène glycol 100 OE, et en particulier celui comprenant un tel mélange en proportion pondérale 50/50, commercialisé sous la dénomination Arlacel 165 par la société Croda.

La composition selon l'invention comprend un polycondensat d'oxyde d'éthylène et d'oxyde de propylène, et plus particulièrement un copolymère consistant en des blocs polyéthylène glycol et polypropylène glycol, comme par exemple les polycondensats tribloc polyéthylène glycol / polypropylène glycol / polyéthylène glycol. Ces polycondensats tribloc ont par exemple la structure chimique suivante :

H-(O-CH₂-CH₂)ₐ-(O-CH(CH₃)-CH₂)b-(O-CH₂-CH₂)ₐ-OH,

formule dans laquelle a va de 2 à 150, et b va de 1 à 100 ; de préférence a va de 10 à 130 et b va de 20 à 80.

Le polycondensat d'oxyde d'éthylène et d'oxyde de propylène a de préférence un poids moléculaire moyen en poids allant de 1000 à 15000, et de mieux allant de 1500 à 15000, et en particulier allant de 1500 à 10000, et encore mieux allant de 1500 à 5000.

Avantageusement, ledit polycondensat d'oxyde d'éthylène et d'oxyde de propylène a une température de trouble, à 10 g/l en eau distillée, supérieure ou égale à 20 °C, de préférence supérieure ou égale à 60 °C. La température de trouble est mesurée selon la norme ISO 1065.

Comme polycondensat d'oxyde d'éthylène et d'oxyde de propylène utilisable selon l'invention, on peut citer les polycondensats tribloc polyéthylène glycol / polypropylène glycol / polyéthylène glycol vendus sous les dénominations "SYNPERONIC" comme les « SYNPERONIC^{®} PE/F32 » (nom INCI : POLOXAMER 108), « SYNPERONIC^{®} PE/F108 » (nom INCI : POLOXAMER 338), "SYNPERONIC^{®} PE/ L44" (nom INCl : POLOXAMER 124) , « SYNPERONIC^{®} PE/L42 (nom INCI : POLOXAMER 122), "SYNPERONIC^{®} PE/F127 " (nom INCI : POLOXAMER 407), « SYNPERONIC^{®} PE/F88 » (nom INCI : POLOXAMER 238), « SYNPERONIC^{®} PE/L64 » (nom INCI : POLOXAMER 184) par la société CRODA , ou encore « LUTROL^{®} F68 » (nom INCI : POLOXAMER 188) par la société BASF.

Le polycondensat d'oxyde d'éthylène et d'oxyde de propylène peut être présent dans la composition selon l'invention en une teneur allant de 0,01 % à 5 % en poids, par rapport au poids total de la composition, de préférence allant de 0,05 % à 3 % en poids, et préférentiellement allant de 0,05 % à 1 % en poids.

La composition selon l'invention peut comprendre un alcool gras ayant de 12 à 22 atomes de carbone, notamment ayant de 14 à 18 atomes de carbone. Un tel alcool gras peut être choisi par exemple parmi l'alcool laurylique, l'alcool cétylique, l'alcool stéarylique. Cet alcool gras peut être présent dans la composition selon l'invention en une teneur allant de 0,05 % à 10 % en poids, par rapport au poids total de la composition, de préférence allant de 0,1 à 5 % en poids, et préférentiellement allant de 1 % à 5 % en poids.

La composition peut également comprendre un acide gras ayant de 12 à 22 atomes de carbone, notamment de 14 à 18 atomes de carbone. Un tel acide gras peut être choisi parmi l'acide laurique, l'acide myristique, l'acide cétylique (ou acide palmitique), l'acide stéarylique, l'alcool cétylstéarylique.
Cet acide gras peut être présent dans la composition selon l'invention en une teneur allant de 0,05 % à 10 % en poids, par rapport au poids total de la composition, de préférence allant de 0,1 à 5 % en poids, et préférentiellement allant de 1 % à 5 % en poids.

La composition selon l'invention peut comprendre en outre un tensioactif anionique choisi parmi les sels alcalins de cétylphosphate. Les sels alcalins sont par exemple les sels de sodium, les sels de potassium. Le tensioactif ionique est de préférence le cétylphosphate de potassium.

On peut notamment utiliser le sel monopotassique de phosphate de monocétyle (nom INCI : potassium cetyl phosphate) vendu sous la dénomination « AMPHISOL K » par la société DSM Nutritional products.

Ce tensioactif anionique permet d'améliorer la stabilité de la composition à haute température (45 °C) pendant 2 mois.

Le tensioactif anionique peut être présent dans la composition selon l'invention en une teneur allant de 0,05 % à 5 % en poids, par rapport au poids total de la composition, de préférence allant de 0,5 à 3 % en poids, et préférentiellement allant de 0,1 % à 3 % en poids.

La composition selon l'invention peut comprendre un gélifiant hydrophile permettant d'épaissir la phase aqueuse de la composition.

Le gélifiant hydrophile peut être choisi par exemple parmi :
(i) les polymères carboxyvinyliques (comme les polymères d'acide acrylique, éventuellement réticulé), tels que les produits commercialisés sous les dénominations Carbopol (nom INCI : Carbomer) par la société Goodrich ;
(ii) les polyacrylamides et les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination « Hostacerin AMPS » (nom INCI : ammonium polyacryloyl-dimethyltaurate) ; les copolymères anioniques réticulés d'acrylamide et d'AMPS, se présentant sous la forme d'une émulsion, tels ceux commercialisés sous le nom de SEPIGEL 305 (nom C.T.F.A. : Polyacrylamide / C₁₃-₁₄ Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ; les copolymères anioniques réticulés d'acide acrylique et d'AMPS, se présentant sous la forme d'une émulsion, tels ceux commercialisés sous le nom de SIMULGEL EG (nom CTFA : Sodium acrylate/sodium acryloyldimethyltaurate copolymer / isohexadecane / Polysorbate 80) ; les copolymères acide 2-acrylamido 2-méthylpropane sulfonique /méthacrylate d'alcool en C12-C14 éthoxylé (ARISTOFLEX LNC de chez Clariant), les copolymères acide 2-acrylamido 2-méthylpropane sulfonique / méthacrylate de stéaryle éthoxylé (ARISTOFLEX HMS et ARISTOFLEX SNC de chez Clariant) ;
(iii) les polysaccharides comme les gommes de xanthane, les gomme de guar, les alginates, les polymères de celluloses comme l'hydroxyéthyl cellulose, l'hydroxypropyl cellulose, la carboxyméthyl cellulose ;
(iv) les composés inorganiques tels que les smectites, les hectorites modifiées ou non telles que les produits BENTONE commercialisés par la société Rheox, les produits LAPONITE commercialisés par la société Southern Clay Products, le produit VEEGUM HS commercialisé par la société R.T.Vanderbilt ; et leurs mélanges.

Parmi ces gélifiants hydrophiles, on choisira plus particulièrement les polysaccharides décrits précédemment et en particulier la gomme de xanthane.

Le gélifiant hydrophile peut être présent dans la composition selon l'invention en une teneur allant de 0,01 % à 10 % en poids, par rapport au poids total de la composition, de préférence allant de 0,1 % à 5 % en poids, et préférentiellement allant de 0,1 % à 3 % en poids.

La composition selon l'invention comprend une phase aqueuse.

La composition peut comprendre de l'eau en une teneur allant de 20 % à 95 % en poids, par rapport au poids total de la composition, de préférence allant de 30 % à 90 % en poids, et préférentiellement allant de 40 % à 70 % en poids.

L'eau peut être une eau florale telle que l'eau de bleuet et/ou une eau minérale telle que l'eau de VITTEL, l'eau de LUCAS ou l'eau de LA ROCHE POSAY et/ou une eau thermale.

La composition peut comprendre en outre un solvant organique miscible à l'eau à la température ambiante (25 °C) notamment choisi parmi les monoalcools ayant de 2 à 6 atomes de carbone tels que l'éthanol, l'isopropanol ;
les polyols ayant notamment de 2 à 20 atomes de carbones, de préférence ayant de 2 à 10 atomes de carbone, et préférentiellement ayant de 2 à 6 atomes de carbones, tels que la glycérine, le propylène glycol, le butylène glycol, le pentylène glycol, l'hexylène glycol, le dipropylène glycol, le diéthylène glycol ;
les éthers de glycol (ayant notamment de 3 à 16 atomes de carbone) tels que les alkyl(C₁-C₄)éther de mono, di- ou tripropylène glycol, les alkyl(C₁-C₄)éthers de mono, diou triéthylène glycol ;
et leurs mélanges.

Avantageusement, la phase aqueuse peut comprendre de l'éthanol.

La composition selon l'invention peut comprendre un solvant organique miscible à l'eau à la température ambiante, notamment un polyol, en une teneur allant de 1 % à 20 % en poids, par rapport au poids total de la composition, et de préférence allant de 3 % à 15 % en poids.

Avantageusement, la composition selon l'invention a un pH allant de 3,0 à 8,0 , de préférence allant de 3,5 à 7,0, préférentiellement allant de 3,5 à 6,0, et plus préférentiellement allant de 3,5 à 5,5.

L'émulsion selon l'invention comprend également une phase huileuse.

Comme huiles plus particulièrement utilisables dans la composition de l'invention, on peut citer par exemple :
- les huiles hydrocarbonées d'origine animale, telles que le perhydrosqualène (ou squalane) ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle (nom INCI : Isopropyl Lauroyl sarcosinate) commercialisé sous la dénomination Eldew SL 205 par la société Ajinomoto ;
- les hydrocarbures linéaires ou ramifiés, d'origine minérale ou synthétique, tels que les huiles minérales (mélange d'huiles hydrocarbonées dérivées du pétrole ; nom INCI : Mineral oil), les huiles de paraffine, volatiles ou non, et leurs dérivés, la vaseline, les polydécènes, l'isohexadecane, l'isododecane, l'isoparaffine hydrogéné tel que l'huile de Parléam® commercialisée par la société NOF Corporation (nom INCI ; Hydrogenated Polyisobutene) ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclopentasiloxane et la cyclohexadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthylsiloxysilicates, et les polyméthylphénylsiloxanes ;
- les huiles fluorées telles que celles partiellement hydrocarbonées et/ou siliconées comme celles décrites dans le document JP-A-2-295912 ;
- les éthers tels que l'éther dicaprylique (nom CTFA : Dicaprylyl ether) ; et les benzoates d'alcools gras en C₁₂-C₁₅ (Finsolv TN de FINETEX) ;
- leurs mélanges.

L'huile peut être présente dans la composition selon l'invention en une teneur allant de 1 % à 50 % en poids, par rapport au poids total de la composition, de préférence allant de 5 % à 40 % en poids, et préférentiellement allant de 5 % à 30 % en poids.

La phase huileuse de l'émulsion peut comprendre d'autres corps gras tels que les cires ; les gommes telles que les gommes de silicone (diméthiconol) ; les résines de silicone, et leurs mélanges.

La composition selon l'invention peut comprendre au moins un agent photoprotecteur organique actif dans l'UVA et/ou l'UVB (absorbeurs), hydrosolubles ou liposolubles ou bien insolubles dans les solvants cosmétiques couramment utilisés.

La composition selon l'invention présentant une bonne stabilité, elle est appropriée pour la formulation de filtres UV organiques : les filtres UV incorporés dans la composition ne sont pas dégradés en présence de composé d'acide ascorbique.

Les filtres organiques sont notamment choisis parmi les anthranilates ; les dérivés cinnamiques ; les dérivés de dibenzoylméthane ; les dérivés ascorbiques, les dérivés du camphre ; les dérivés de triazine autres que ceux de l'invention tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US 5,237,071, US 5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Avantageusement, on utilise un filtre protecteur organique non ionique.

L'agent photoprotecteur peut être présent dans la composition selon l'invention en une teneur allant de 0,01 % à 30% en poids, par rapport au poids total de la composition, de préférence allant de 0,1 à 25% en poids , et préférentiellement allant de 0,1 % à 20 % en poids.

La composition selon l'invention peut comprendre en outre des charges.

Par charges, il faut comprendre des particules de toute forme, incolores ou blanches, minérales ou organiques, insolubles dans le milieu de la composition quelle que soit la température à laquelle la composition est fabriquée, et qui ne colorent pas la composition.

Les charges peuvent être de toute forme, plaquettaires, sphériques ou oblongues, quelle que soit la forme cristallographique (par exemple feuillet, cubique, hexagonale, orthorombique, etc) . On peut citer : le talc, le mica, la silice, le kaolin, de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthanes, les charges composites, les microsphères de silice creuses, et les microcapsules de verre ou de céramique.

Les charges peuvent être présentes dans la composition en une teneur allant de 0,1 % à 15 % en poids, et de préférence allant de 0,1 % à 10 % en poids, et préférentiellement de 0,1 % à 5 % en poids, par rapport au poids total de la composition.

La composition selon l'invention peut comprendre en outre un actif choisi parmi les agents desquamants capables d'agir, soit en favorisant l'exfoliation, soit sur les enzymes impliquées dans la desquamation ou la dégradation des cornéodesmosomes, les agents hydratants, les agents dépigmentants ou pro-pigmentants, les agents anti-glycation, les inhibiteurs de NO-synthase, les inhibiteurs de 5α-réductase, les inhibiteurs de lysyl et/ou prolyl hydroxylase, les agents stimulant la synthèse de macromolécules dermiques ou épidermiques et/ou empêchant leur dégradation, les agents stimulant la prolifération des fibroblastes ou des kératinocytes et/ou la différenciation des kératinocytes, les agents myorelaxants, les agents anti-microbiens, les agents tenseurs, les agents anti-pollution ou anti-radicalaires, les agents anti-inflammatoires, les actifs lipolytiques ou ayant une activité favorable, directe ou indirecte, sur la diminution du tissu adipeux, les agents agissant sur la microcirculation, et les agents agissant sur le métabolisme énergétique des cellules.

La composition selon l'invention est en particulier destinée à un usage topique, notamment cosmétique ou dermatologique.

De façon connue, la composition cosmétique ou dermatologique de l'invention peut contenir également des adjuvants habituels dans le domaine cosmétique ou dermatologique, tels que les conservateurs, les parfums, les bactéricides, les absorbeurs d'odeur, les matières colorantes, les sels, les tensioactifs, les épaississants, les bases. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse ou dans la phase aqueuse.

La composition selon l'invention peut être appliquée sur la peau ou les lèvres, suivant l'usage auquel elle est destinée. Elle peut ainsi être utilisée dans un procédé de traitement cosmétique de la peau, comprenant l'application de la composition selon l'invention sur la peau, par exemple en vue de la tonifier, de la régénérer, de lisser les ridules de la peau, et/ou pour lutter contre le vieillissement cutané, contre les méfaits des rayonnements UV et/ou pour renforcer les tissus cutanés contre les agressions de l'environnement.

En variante, la composition selon l'invention peut être utilisée pour la fabrication d'une préparation dermatologique.

La composition peut être une composition de soin, notamment être un produit de soin de la peau tels qu'une base de soin pour la peau, une crème de soin (crème de jour, de nuit, anti-rides), une base de maquillage ; une composition de soin pour les lèvres (baume à lèvres) ; une composition de protection solaire ou autobronzante.

La composition peut également être une composition de maquillage, notamment de maquillage de la peau ou des lèvres. En particulier, la composition de maquillage peut être un fond de teint, un fard à joue, un fard à paupière, un produit anti-cernes, un produit de maquillage du corps.

Avantageusement, la composition est une composition non rincée.

L'émulsion selon l'invention peut être préparée selon le mode opératoire général suivant : Mélanger les constituants de la phase aqueuse en chauffant à une température d'environ 70 °C. Mélanger par ailleurs les huiles, l'actif de formule (I) et les tensioactifs en chauffant à une température d'environ 80 °C. Verser la phase grasse dans la phase aqueuse, à une température d'environ 70 °C puis agiter pendant 10 minutes à l'aide d'une turbine, à grande vitesse. Refroidir l'émulsion obtenue à environ 60 °C. Ajouter ensuite les épaississants, puis agiter à nouveau pendant 10 minutes. Introduire ensuite les autres actifs éventuellement.

L'invention sera maintenant illustrée à l'aide des exemples non limitatifs suivants.

### Exemple 1 comparatif:

On a préparé une composition de soin du visage sous forme d'émulsion huile-dans-eau selon l'invention (exemple 1) ayant la composition suivante :
- Mélange de monostéarate de glycéryle et de stéarate de polyéthylèneglycol (100 OE) (ARLACEL 165 FL de chez CRODA) 1,6 g
- Monostéarate de sorbitane oxyéthyléné (20 OE) (TWEEN 60 de chez Croda) 0,7 g
- Alcool cétylique 1 g
- Acide stéarique 0,5 g
- Huile d'amande d'abricot 5 g
- Polydimethylsiloxane 10 cst 7,0 g
- Isoparaffine hydrogénée (Parleam de chez NOF Corporation) 2,0 g

- 4-(1-phényléthyl)-1,3-dihydroxybenzène 0,3 g

- Acide poly acrylamidométhyl propane sulfonique neutralisé partiellement à l'ammoniaque et hautement réticulé (Hostacerin AMPS de chez Clariant) 1,0 g
- Gomme de xanthane 0,3 g
- Microsphères de silice (SB700 de chez Myoshi Kasei) 3,0 g

- Copolymère bloc OE-OP-OE (128 EO/54 OP/128 OE) (Synperonic^{®} PE/F108 de chez CRODA) 0,4 g
- Glycérol 5 g
- Sel disodique de l'acide éthylène diamine tétracétique 0,2 g
- Conservateurs qs
- Eau qsp 100 g

On a également préparé une composition similaire à la composition 1 mais contenant en plus 1,5 g de cétyl phosphate de potassium (composition 1a selon l'invention) (quantité ajoutée en supprimant le même poids d'eau).

On a également préparé une composition similaire à la composition 1 mais ne contenant pas de Copolymère bloc OE-OP-OE (Synperonic^{®} PE/F108 de chez CRODA) (composition 1b ne faisant pas partie de l'invention) (quantités supprimées remplacées par le même poids d'eau).

Les émulsions des exemples 1 et 1a présentent une bonne stabilité après 24 heures à 55 °C et également après 2 mois à 45 °C. En observation au microscope, l'émulsion obtenue est fine et serrée.

La composition est appliquée sur le visage en utilisation quotidienne de jour.

L'émulsion comparative de l'exemple 1b est instable après 24 heures à 55 °C et après conservation pendant 2 mois à 45 °C : l'émulsion, observée au microscope, est plus grossière, plus dégradée. A l'oeil nu, on observe un déphasage avec relargage en surface de la phase huileuse.

### Exemple 2 comparatif:

On a préparé une composition de soin du visage sous forme d'émulsion huile-dans-eau ayant la composition suivante :
- Mélange de monostéarate de glycéryle et de stéarate de polyéthylèneglycol (100 OE) (ARLACEL 165 FL de chez CRODA) 0,5 g
- Acide stéarique 1 g
- Alcool cétylique 0,5 g
- Stéarate d'isocétyle 5 g
- 4-(1-phényléthyl)-1,3-dihydroxybenzène 0,5 g
- Drometrizole trisiloxane (Silitrizole de chez Rhodia) 1 g
- Triglycérides d'acide caprylique/caprique (60/40) 3 g
- Acide n-octanoyl-5 salicylique 0,3 g

- Acétate de tocophérol 1 g
- Ascorbyl glucoside 0,05 g
- Gomme de xanthane (Rhodicare XC de chez Rhodia) 0,15 g
- copolymère acrylamide/acrylamido 2-méthyl propane sulfonate de sodium en émulsion inverse à 40% dans isoparaffine/eau (Sepigel 305 de chez Seppic) 1,8 g
- Hexamétaphosphate de sodium 0,05 g
- Sel disodique de l'acide éthylène diamine tétracétique 0,05 g
- Copolymère bloc OE-OP-OE (Synperonic^{®} PE/F108 de chez CRODA) 0,4 g
- Glycérol 7 g
- Butylène glycol 3 g
- Conservateurs qs
- Parfum qs
- Eau qsp 100 g

On a également préparé une composition similaire à la composition 3 mais contenant en plus 1,5 g de cétyl phosphate de potassium (composition 3a selon l'invention) (quantité ajoutée en supprimant le même poids d'eau).

On a également préparé une composition similaire à la composition 3 mais ne contenant pas de copolymère bloc OE-OP-OE (Synperonic^{®} PE/F108 de chez CRODA) et de cétyl phosphate de potassium (composition 3b ne faisant pas partie de l'invention) (quantités supprimées remplacées par le même poids d'eau).

Les émulsions des exemples 3 et 3a présentent une bonne stabilité après 24 heures à 55 °C. En observation au microscope, l'émulsion obtenue est fine et serrée. L'émulsion 3a présente en plus une bonne stabilité après 2 mois à 45 °C.

La composition est appliquée sur le visage en utilisation quotidienne de jour.

L'émulsion comparative de l'exemple 3b est instable après conservation pendant 2 mois à 45 °C : l'émulsion, observée au microscope, est plus grossière, plus dégradée. A l'oeil nu, on observe un déphasage avec relargage en surface de la phase huileuse.

## Revendications

1. Composition sous forme d'émulsion huile-dans-eau comprenant :
- un ester d'acides gras en C₁₆-C₂₂ comportant de 8 à 100 unités d'oxyde éthylène;
- un tensioactif additionnel choisi parmi les esters d'acide gras en C₁₆-C₂₂ et de sorbitan et les esters d'acide gras en C₁₆-C₂₂ et de glycéryle ;
- un polycondensat d'oxyde d'éthylène et d'oxyde de propylène consistant en des blocs polyéthylène glycol et polypropylène glycol ;
- un dérivé diphényl-méthane hydroxylé de formule (I) : 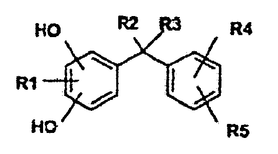 dans laquelle :
- R1 est choisi parmi un atome d'hydrogène, un groupement méthyle, une chaîne alkyle linéaire ou ramifiée, saturée ou insaturée, ayant de 2 à 4 atomes de carbone, un groupement -OH, et un halogène,
- R2 est choisi parmi un atome d'hydrogène, un groupement méthyle, une chaîne alkyle linéaire ou ramifiée, saturée ou insaturée, ayant de 2 à 5 atomes de carbone,
- R3 est choisi parmi un groupement méthyle ou une chaîne alkyle linéaire ou ramifiée, saturée ou insaturée, ayant de 2 à 5 atomes de carbone,
- R4 et R5 sont indépendemment l'un de l'autre choisi parmi un atome d'hydrogène, un groupement méthyle, une chaîne alkyle linéaire ou ramifiée, saturée ou insaturée, ayant de 2 à 5 atomes de carbone, un groupement -OH ou un halogène.

2. Composition selon la revendication précédente, **caractérisée par le fait que** le dérivé diphényl-méthane hydroxylé a la fomule (II) suivante :

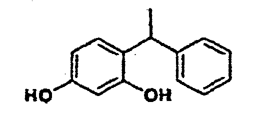

3. Composition selon l'une quelconque des revendications précédentes, **caractérisée par le fait que** le dérivé diphényl-méthane hydroxylé de formule (I) ou (II) est présent en une teneur allant de 0,01 % à 5 % en poids, par rapport au poids total de la composition, de préférence allant de 0,01 % à 2 % en poids, et préférentiellement allant de 0,01 % à 1 % en poids.

4. Composition selon l'une quelconque des revendications précédentes, **caractérisée par le fait que** l'ester d'acide gras et de polyéthylène glycol est choisi parmi les stéarates de polyéthylène glycol comportant de 10 à 80 unités d'oxyde d'ethylene.

5. Composition selon l'une quelconque des revendications précédentes, **caractérisée par le fait que** l'ester d'acide gras en C₁₆-C₂₂ de polyéthylène glycol comprend de 20 à 40 unités d'oxyde d'éthylène.

6. Composition selon l'une quelconque des revendications précédentes, **caractérisée par le fait qu'**elle comprend un ester d'acide gras en C₁₆-C₂₂ et de sorbitan choisi parmi les stéarates de sorbitan.

7. Composition selon l'une quelconque des revendications précédentes, **caractérisée par le fait qu'**elle comprend un ester d'acide gras en C₁₆-C₂₂ et de glycéryle choisi parmi les stéarates de glycéryle.

8. Composition selon l'une quelconque des revendications précédentes, **caractérisée par le fait qu'**elle comprend un mélange de stéarate de glycéryle et de monostéarate de polyéthylène glycol 100 OE.

9. Composition selon l'une quelconque des revendications précédentes, **caractérisée par le fait qu'**elle comprend un polycondensat tribloc polyéthylène glycol / polypropylène glycol / polyéthylène glycol.

10. Composition selon l'une quelconque des revendications précédentes, **caractérisée par le fait que** le polycondensat d'oxyde d'éthylène et d'oxyde de propylène a un poids moléculaire moyen en poids allant de 1000 à 15000, et de mieux allant de 2000 à 13000.

11. Composition selon l'une quelconque des revendications précédentes, **caractérisée par le fait qu'**elle comprend un gélifiant hydrophile.

12. Composition selon la revendication précédente, **caractérisée par le fait que** le gélifiant hydrophile est un polysaccharide.

13. Composition selon l'une quelconque des revendications précédentes, **caractérisée par le fait qu'**elle comprend au moins un adjuvant cosmétique ou dermatologique choisi parmi les filtres UV, les charges, les conservateurs, les parfums, les bactéricides, les absorbeurs d'odeur, les matières colorantes, les sels, les tensioactifs, les épaississants, les bases.

14. Procédé non thérapeutique de soin ou de maquillage des matières kératiniques comprenant l'application sur les matières kératiniques d'une composition selon l'une quelconque des revendications précédentes.

## Claims

1. Composition in the form of an oil-in-water emulsion comprising:
- a C₁₆-C₂₂ fatty acid ester comprising from 8 to 100 ethylene oxide units;
- an additional surfactant chosen from esters of C₁₆-C₂₂ fatty acid and of sorbitan and esters of C₁₆-C₂₂ fatty acid and of glycerol;
- a polycondensate of ethylene oxide and of propylene oxide consisting of polyethylene glycol and polypropylene glycol blocks;
- a hydroxylated diphenylmethane derivative of formula (I) : 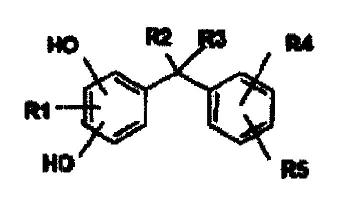 in which:
- R1 is chosen from a hydrogen atom, a methyl group, a saturated or unsaturated and linear or branched alkyl chain having from 2 to 4 carbon atoms, an OH group and a halogen,
- R2 is chosen from a hydrogen atom, a methyl group or a saturated or unsaturated and linear or branched alkyl chain having from 2 to 5 carbon atoms,
- R3 is chosen from a methyl group or a saturated or unsaturated and linear or branched alkyl chain having from 2 to 5 carbon atoms,
- R4 and R5 are chosen, independently of one another, from a hydrogen atom, a methyl group, a saturated or unsaturated and linear or branched alkyl chain having from 2 to 5 carbon atoms, an -OH group or a halogen.

2. Composition according to the preceding claim, **characterized in that** the hydroxylated diphenylmethane derivative has the following formula (II):

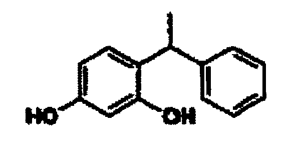

3. Composition according to either of the preceding claims, **characterized in that** the hydroxylated diphenylmethane derivative of formula (I) or (II) is present in a content ranging from 0.01% to 5% by weight, with respect to the total weight of the composition, preferably ranging from 0.01% to 2% by weight and preferentially ranging from 0.01% to 1% by weight.

4. Composition according to any one of the preceding claims, **characterized in that** the ester of fatty acid and of polyethylene glycol is chosen from polyethylene glycol stearates comprising from 10 to 80 ethylene oxide units.

5. Composition according to any one of the preceding claims, **characterized in that** the ester of C₁₆-C₂₂ fatty acid and of polyethylene glycol comprises from 20 to 40 ethylene oxide units.

6. Composition according to any one of the preceding claims, **characterized in that** it comprises an ester of C₁₆-C₂₂ fatty acid and of sorbitan chosen from sorbitan stearates.

7. Composition according to any one of the preceding claims, **characterized in that** it comprises an ester of C₁₆-C₂₂ fatty acid and of glycerol chosen from glyceryl stearates.

8. Composition according to any one of the preceding claims, **characterized in that** it comprises a mixture of glyceryl stearate and of polyethylene glycol 100 EO monostearate.

9. Composition according to any one of the preceding claims, **characterized in that** it comprises a polyethylene glycol/oolypropylene glycol/polyethylene glycol triblock polycondensate.

10. Composition according to any one of the preceding claims, **characterized in that** the polycondensate of ethylene oxide and of propylene oxide has a weight-average molecular weight ranging from 1000 to 15 000 and better still ranging from 2000 to 13 000.

11. Composition according to any one of the preceding claims, **characterized in that** it comprises a hydrophilic gelling agent.

12. Composition according to the preceding claim, **characterized in that** the hydrophilic gelling agent is a polysaccharide.

13. Composition according to any one of the preceding claims, **characterized in that** it comprises at least one cosmetic or dermatological adjuvant chosen from UV screening agents, fillers, preservatives, fragrances, bactericides, odour absorbers, colouring materials, salts, surfactants, thickeners or bases.

14. Non-therapeutic method for caring for or making up keratinous substances, comprising the application, to the keratinous substances, of a composition according to any one of the preceding claims.

## Patentansprüche

1. Zusammensetzung in Form einer Öl-in-Wasser-Emulsion, umfassend:
- einen Ester von C₁₆-C₂₂-Fettsäuren mit 8 bis 100 Ethylenoxid-Einheiten;
- ein zusätzliches Tensid, das unter Estern von C₁₆-C₂₂-Fettsäure und Sorbitan und Estern von C₁₆-C₂₂-Fettsäure und Glycerin ausgewählt ist;
- ein Polykondensat von Ethylenoxid und Propylenoxid, das aus Polyethylenglykol- und Polypropylenglykolblöcken besteht;
- ein hydroxyliertes Diphenylmethanderivat der Formel (I): 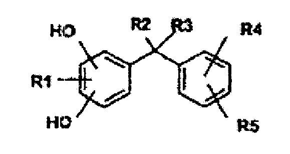 worin:
- R1 unter einem Wasserstoffatom, einer Methylgruppe, einer linearen oder verzweigten, gesättigten oder ungesättigten Alkylkette mit 2 bis 4 Kohlenstoffatomen, einer -OH-Gruppe und einem Halogen ausgewählt ist,
- R2 unter einem Wasserstoffatom, einer Methylgruppe und einer linearen oder verzweigten, gesättigten oder ungesättigten Alkylkette mit 2 bis 5 Kohlenstoffatomen ausgewählt ist,
- R3 unter einer Methylgruppe und einer linearen oder verzweigten, gesättigten oder ungesättigten Alkylkette mit 2 bis 5 Kohlenstoffatomen ausgewählt ist,
- R4 und R5 unabhängig voneinander unter einem Wasserstoffatom, einer Methylgruppe, einer linearen oder verzweigten, gesättigten oder ungesättigten Alkylkette mit 2 bis 5 Kohlenstoffatomen, einer -OH-Gruppe oder einem Halogen ausgewählt sind.

2. Zusammensetzung nach dem vorhergehenden Anspruch, dadurch gekenntzeichnet, dass das hydroxylierte Diphenylmethanderivat die folgende Formel (II) aufweist:

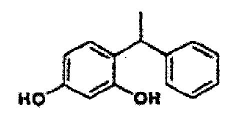

3. Zusammensetzung nach einem der vorhergehenden Ansprüche, **dadurch gekennzeichnet, dass** das hydroxylierte Diphenylmethanderivat der Formel (I) oder (II) in einem Gehalt von 0,01 bis 5 Gew.-%, bezogen auf das Gesamtgewicht der Zusammensetzung, vorzugsweise von 0,01 bis 2 Gew.-% und bevorzugt von 0,01 bis 1 Gew.-% vorliegt.

4. Zusammensetzung nach einem der vorhergehenden Ansprüche, **dadurch gekennzeichnet, dass** der Ester von Fettsäure und Polyethylenglykol unter Polyethylenglykolstearaten mit 10 bis 80 Ethylenoxid-Einheiten ausgewählt ist.

5. Zusammensetzung nach einem der vorhergehenden Ansprüche, **dadurch gekennzeichnet, dass** der Ester von C₁₆-C₂₂-Fettsäure und Polyethylenglykol 20 bis 40 Ethylenoxid-Einheiten umfasst.

6. Zusammensetzung nach einem der vorhergehenden Ansprüche, **dadurch gekennzeichnet, dass** sie einen Ester von C₁₆-C₂₂-Fettsäure und Sorbitan, der unter Sorbitanstearaten ausgewählt ist, umfasst.

7. Zusammensetzung nach einem der vorhergehenden Ansprüche, **dadurch gekennzeichnet, dass** sie einen Ester von C₁₆-C₂₂-Fettsäure und Glycerin, der unter Glycerylstearaten ausgewählt ist, umfasst.

8. Zusammensetzung nach einem der vorhergehenden Ansprüche, **dadurch gekennzeichnet, dass** sie eine Mischung von Glycerylstearat und Polyethylenglykol-100-EO-monostearat umfasst.

9. Zusammensetzung nach einem der vorhergehenden Ansprüche, **dadurch gekennzeichnet, dass** sie ein Polyethylenglykol/Polypropylenglykol/Polyethylenglykol-Dreiblockpolykondensat umfasst.

10. Zusammensetzung nach einem der vorhergehenden Ansprüche, **dadurch gekennzeichnet, dass** das Polykondensat von Ethylenoxid und Propylenoxid ein gewichtsmittleres Molekulargewicht von 1000 bis 15.000 und noch besser 2000 bis 13.000 aufweist.

11. Zusammensetzung nach einem der vorhergehenden Ansprüche, **dadurch gekennzeichnet, dass** sie ein hydrophiles Geliermittel umfasst.

12. Zusammensetzung nach dem vorhergehenden Anspruch, **dadurch gekennzeichnet, dass** es sich bei dem hydrophilen Geliermittel um ein Polysaccharid handelt.

13. Zusammensetzung nach einem der vorhergehenden Ansprüche, **dadurch gekennzeichnet, dass** sie mindestens einen kosmetischen oder dermatologischen Hilfsstoff, der unter UV-Filtern, Füllstoffen, Konservierungsstoffen, Parfümen, Bakteriziden, Geruchsabsorbern, farbgebenden Substanzen, Salzen, Tensiden, Verdickern und Basen ausgewählt ist, umfasst.

14. Nichttherapeutisches Verfahren zur Pflege oder zum Makeup von Keratinmaterialien, bei dem man auf die Keratinmaterialien eine Zusammensetzung nach einem der vorhergehenden Ansprüche aufbringt.
